(19)

Europäisches
Patentamt
European
Patent Office
Office européen
des brevets

(11) **EP 4 613 861 A1**

(12) **EUROPEAN PATENT APPLICATION**
published in accordance with Art. 153(4) EPC

(43) Date of publication:
**10.09.2025 Bulletin 2025/37**

(21) Application number: 23884938.4

(22) Date of filing: **31.10.2023**

(51) International Patent Classification (IPC):
*C12N 15/12* (2006.01)  *A61K 48/00* (2006.01)
*C12N 15/90* (2006.01)

(52) Cooperative Patent Classification (CPC):
**A61K 45/06; A61K 48/00; A61P 7/04; A61P 7/10;
C07K 14/435; C12N 5/10; C12N 15/66;
C12N 15/864; C12N 15/90**

(86) International application number:
**PCT/CN2023/128560**

(87) International publication number:
**WO 2024/094009 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022  CN 202211351080**

(71) Applicant: **Suzhou Heguang Kehui Biotechnology
Co., Ltd
Suzhou, Jiangsu 215000 (CN)**

(72) Inventors:
• **YU, Tenghui
Suzhou, Jiangsu 215000 (CN)**

• **YANG, Zhou
Suzhou, Jiangsu 215000 (CN)**
• **ZHANG, Jianghong
Suzhou, Jiangsu 215000 (CN)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

Remarks:
The complete document including Reference
Table(s) and the Sequence Listing(s) can be
downloaded from the EPO website

(54) **EXPRESSION CASSETTE FOR TARGET GENE AND USE THEREOF**

(57)    The present invention provides an expression cassette for a target gene and use thereof. Specifically, the present invention provides an expression cassette having elements such as HCR, DSE, TPL and eMlp, an encoding nucleic acid thereof, an expression vector thereof, a host cell thereof, a pharmaceutical composition thereof, a gene delivery system thereof, and use thereof. The present invention further provides a C1-INH protein-encoding nucleic acid molecule and use thereof.

EP 4 613 861 A1

**Description**

**Technical field**

The present invention belongs to the field of biotechnology. Specifically, it relates to an expression cassette for a target gene and uses thereof.

**Background**

Gene therapy is an effective way to treat genetic diseases. It involves transducing exogenous normal genes (i.e., target genes) into target cells to correct or compensate for diseases caused by defective or abnormal genes, thus achieving therapeutic goals. Gene therapy primarily focuses on diseases that pose severe threats to human health, including: genetic diseases (such as hemophilia, cystic fibrosis, familial hypercholesterolemia, hereditary angioedema, etc.), malignant tumors, cardiovascular diseases, and infectious diseases (such as AIDS, rheumatoid diseases, etc.).

In gene therapy, how to effectively express target genes in target cells and achieve the desired expression effect is of vital importance. Hereditary angioedema can be taken as an example, which is caused by a deficiency of C1-INH protein. Normally, the content of C1-INH protein in the blood should reach 160 $\mu$g/ml-320 $\mu$g/ml. However, previous clinical trials have shown that a blood concentration of at least 112 $\mu$g/ml of C1-INH is required for a curative effect. Such a high content poses a considerable challenge for current gene therapy methods. Therefore, there is an urgent need for an ideal expression cassette or expression vector, which is expected to have various advantages, such as high titer, ability to infect a large number of cells, easy preparation and good reproducibility, ability to specifically enter target cells and integrate into the host chromosome at specific sites, stable existence in the form of an episome, and absence of components that can trigger immune responses, so as to enhance the effectiveness and success rate of gene therapy and reduce side effects.

Therefore, there is a need in this field to develop an expression cassette that can effectively increase the expression level of target genes.

**Summary of the invention**

The purpose of the present invention is to provide an expression cassette for target genes with high expression and low side-effects.

Another purpose of the present invention is to provide a gene delivery system with long-term expression.

Another purpose of the present invention is to provide a novel therapeutic approach with low side-effects that can achieve life-long benefits from a single administration.

In the first aspect of the present invention, is provided an expression cassette having a structure shown in formula I from the 5' to 3' end:

$$Z1\text{-}Z2\text{-}Z3\text{-}Z4\text{-}Z5\text{-}Z6\text{-}Z7\text{-}Z8 \qquad (I)$$

wherein each "-" is independently a bond or a nucleotide linker sequence;
Z1 is an HCR element;
Z2 is a DSE element;
Z3 is a TPL element;
Z4 is an eMlp element;
Z5 is an intron element;
Z6 is absent or is a Kozak sequence;
Z7 is a target gene; and
Z8 is a poly(A) element.

In another preferred embodiment, the sequence of Z1 is selected from the group consisting of: SEQ ID NO: 4, SEQ ID NO: 10, and a combination thereof.

In another preferred embodiment, the sequence of Z1 is as set forth in SEQ ID NO: 10.

In another preferred embodiment, the sequence of Z1 is as set forth in SEQ ID NO: 4.

In another preferred embodiment, the sequence of Z2 is as set forth in SEQ ID NO: 5.

In another preferred embodiment, the sequence of Z3 is selected from the group consisting of: SEQ ID NO: 6, SEQ ID NO: 11, and a combination thereof.

In another preferred embodiment, the sequence of Z3 is as set forth in SEQ ID NO: 11.

In another preferred embodiment, the sequence of Z3 is as set forth in SEQ ID NO: 6.

In another preferred embodiment, the sequence of Z4 is as set forth in SEQ ID NO: 7.

In another preferred embodiment, Z5 is selected from the group consisting of: β-globin intron, SV40 intron, HBB2 intron, VH4 intron, U12 intron, Chi intron, RHD intron, an intron of SRB gene, an intron of Minute Virus of Mice (MVM), a segment of said introns, and a combination thereof.

In another preferred embodiment, Z5 is selected from the group consisting of: SV40 intron or a segment thereof, HBB2 intron or a segment thereof, and a combination thereof.

In another preferred embodiment, Z5 is the SV40 intron.

In another preferred embodiment, the sequence of Z5 is as set forth in SEQ ID NO: 8.

In another preferred embodiment, Z5 is a chimeric intron composed of a segment of the SV40 intron and a segment of the HBB2 intron.

In another preferred embodiment, Z5 comprises the sequence as set forth in SEQ ID NO: 12.

In another preferred embodiment, the target gene is selected from the group consisting of: a normal gene, an antisense gene, a suicide gene, and a combination thereof.

In another preferred embodiment, Z7 is selected from the group consisting of: serpinG1 gene, FIX gene, PAH (phenylketonuria), GBA1 gene (Gaucher's disease), GLA gene (Fabry disease), IDS (mucopolysaccharidosis type II), G6P (favism), GAA (Pompe disease), luciferase gene, CFTR gene (cystic fibrosis), LDLR gene (familial hypercholesterolemia), α-globin gene, β-globin gene (thalassemia), APC gene (familial adenomatous polyposis), SLC26A4 gene, GJB2 gene (congenital deafness), TYR gene, OCA2 gene, TYRP1 gene, SLC45A2 gene (albinism), and a combination thereof.

In another preferred embodiment, the sequence of Z7 is as set forth in SEQ ID NO: 1 or SEQ ID NO: 2; and preferably, the sequence of Z7 is as set forth in SEQ ID NO: 1.

In another preferred embodiment, Z8 is selected from the group consisting of: bGH poly(A), short poly(A), SV40 poly(A), synthetic SPA51 poly(A), human β-globin poly(A), and a combination thereof.

In another preferred embodiment, Z8 is bGH poly(A).

In another preferred embodiment, the sequence of Z8 is selected from the group consisting of: SEQ ID NO: 13, SEQ ID NO: 14, and a combination thereof.

In another preferred embodiment, the sequence of Z8 is as set forth in SEQ ID NO: 14.

In another preferred embodiment, the sequence of Z8 is as set forth in SEQ ID NO: 13.

In another preferred embodiment, the expression cassette has a nucleotide sequence as set forth in SEQ ID NO: 3 or SEQ ID NO: 15.

In another preferred embodiment, the nucleotide sequence of the expression cassette has at least 50% identity, preferably at least 60%, 70%, 80%, 90%, 95%, 99%, or 100% identity with the sequence as set forth in SEQ ID NO: 3 or SEQ ID NO: 15.

In the second aspect of the present invention, is provided a nucleic acid molecule encoding the expression cassette of the first aspect of the present invention.

In another preferred embodiment, the nucleic acid molecule may be a RNA, DNA, or cDNA.

In another preferred embodiment, the sequence of the nucleic acid molecule is as set forth in SEQ ID NO: 3.

In another preferred embodiment, the sequence of the nucleic acid molecule has at least 50% identity, preferably has at least 60%, 70%, 80%, 90%, 95%, 96%, 97%, 98%, 99%, or 100% identity with the sequence as set forth in SEQ ID NO: 3.

In the third aspect of the present invention, is provided an expression vector comprising the nucleic acid molecule of the second aspect of the present invention or the expression cassette of the first aspect of the present invention.

In another preferred embodiment, the expression vector is selected from the group consisting of: DNA, RNA, viral vectors, plasmids, transposons, other gene transfer systems, and combinations thereof. Preferably, the expression vector comprises a viral vector, such as lentivirus, adenovirus, AAV virus, retrovirus, or a combination thereof.

In another preferred embodiment, the expression vector is an AAV vector.

In another preferred embodiment, the expression vector is selected from the group consisting of: pTomo lentiviral vector, plenti, pLVTH, pLJM1, pHCMV, pLBS.CAG, pHR, pLV, etc.

In another preferred embodiment, the expression vector further comprises elements selected from the group consisting of: promoters, WPRE transcriptional enhancer elements, long terminal repeat (LTR) sequences, etc.

In another preferred embodiment, the expression vector comprises one or more promoters operably linked to the polynucleotide or a fragment thereof, enhancer, intron, transcriptional termination signal, polyadenylation sequence, replication origin, selective marker, nucleic acid restriction site, and/or homologous recombination site.

In another preferred embodiment, the promoter is selected from the group consisting of: CB promoter, SV40 promoter, SOX9 promoter, ALB promoter, TBG promoter, ApoA1 promoter, TTR promoter, CAG promoter, AAT promoter, and a combination thereof.

In another preferred embodiment, the intron is selected from the group consisting of: a SV40 intron, VH4 intron, U12 intron, Chi intron, RHD intron, and a combination thereof.

In another preferred embodiment, the expression vector has a structure shown in formula II from the 5' to 3' end:

A1-A2-A3    (II)

wherein each "-" is independently a bond or a nucleotide linker sequence;
A1 is an ITR-L sequence;
A2 is the expression cassette of the first aspect of the present invention; and
A3 is an ITR-R sequence.

In the fourth aspect of the present invention, is provided a host cell comprising the expression vector of the third aspect of the present invention or having the nucleic acid molecule of the second aspect of the present invention integrated into its genome.

In another preferred embodiment, the host cell comprises a prokaryotic or eukaryotic cell.

In another preferred embodiment, the host cell is selected from the group consisting of: *E. coli,* yeast cells, and mammalian cells.

In the fifth aspect of the present invention, is provided a gene delivery system, comprising: the expression cassette of the first aspect of the present invention or the nucleic acid molecule of the second aspect of the present invention, and an AAV capsid protein.

In another preferred embodiment, the AAV capsid protein is a natural AAV capsid protein or an engineered AAV capsid protein.

In another preferred embodiment, the AAV includes but is not limited to the group consisting of: AAV1, AAV2, AAV3, AAV4, AAV5, AAV6, AAV7, AAV8, AAV9, AAV10, AAV11, AAV12, and AAV-DJ.

In another preferred embodiment, the AAV capsid protein is the capsid protein of AAV8, which has the amino acid sequence as set forth in SEQ ID NO: 9.

In the sixth aspect of the present invention, is provided a use of the expression cassette of the first aspect of the present invention, the nucleic acid molecule of the second aspect of the present invention, the expression vector of the third aspect of the present invention, the host cell of the fourth aspect of the present invention, or the gene delivery system of the fifth aspect of the present invention, in the manufacture of a formulation or composition for use as a gene therapy medicament.

In the seventh aspect of the present invention, is provided a pharmaceutical composition, comprising:

(i) the expression cassette of the first aspect of the present invention, the nucleic acid molecule of the second aspect of the present invention, the expression vector of the third aspect of the present invention, the host cell of the fourth aspect of the present invention, or the gene delivery system of the fifth aspect of the present invention, as an active ingredient; and
(ii) a pharmaceutically acceptable carrier, diluent, or excipient.

In another preferred embodiment, component (i) accounts for 0.1-99.9 wt%, preferably 10-99.9 wt%, and more preferably 70-99 wt% of the total weight of the pharmaceutical composition.

In another preferred embodiment, the dosage form of the pharmaceutical composition is selected from the group consisting of: lyophilized dosage forms and liquid dosage forms.

In another preferred embodiment, the dosage form of the pharmaceutical composition is an injection.

In another preferred embodiment, the method of administration of the pharmaceutical composition comprises intravenous injection.

In another preferred embodiment, the pharmaceutical composition is an injectable form for intravenous injection.

In another preferred embodiment, the pharmaceutically acceptable carrier includes but is not limited to: solvents, dispersion media, coatings, antibacterial or antifungal agents, isotonic agents, and absorption-delaying agents, etc.

In another preferred embodiment, the pharmaceutically acceptable carrier is an injection carrier; and preferably, the pharmaceutically acceptable carrier comprises saline, wherein the saline includes but is not limited to: buffered saline, normal saline, phosphate buffer, citrate buffer, acetate buffer, bicarbonate buffer, sucrose solution, salt solution, polysorbate solution, or a combination thereof.

In another preferred embodiment, the pharmaceutically acceptable carrier may further comprise additives, including but not limited to: stabilizers, preservatives, transfection enhancers that facilitate cellular uptake, or combinations thereof.

In another preferred embodiment, the pharmaceutical composition may be used alone or in combination in gene therapy applications.

In another preferred embodiment, the combination use comprises: coadministration with other gene therapy medicaments.

In the eighth aspect of the present invention, is provided a gene therapy method, comprising a step of:

administering the expression vector of the third aspect of the present invention, the gene delivery system of the fifth aspect of the present invention, or the pharmaceutical composition of the seventh aspect of the present invention, to a subject in need thereof.

In another preferred embodiment, the method of administration is intravenous injection.

In another preferred embodiment, the subject in need comprises a human and a non-human mammal.

In another preferred embodiment, the subject in need is a patient with hereditary angioedema or a patient with hemophilia.

In another preferred embodiment, the dosage of the gene delivery system is 6E11 vg/kg to 6E13 vg/kg, preferably 2E12 vg/kg to 4E13 vg/kg, and more preferably 6E12 vg/kg to 2E13 vg/kg.

In the ninth aspect of the present invention, is provided a nucleic acid molecule encoding the serpinG1 gene, the nucleotide sequence of which has at least 87% identity, preferably at least 88%, 89%, 90%, 91%, 92%, 93%, 94%, 95%, 96%, 97%, 98%, 99%, or 100% identity to the sequence as set forth in SEQ ID NO: 1.

In the tenth aspect of the present invention, is provided an expression vector comprising the nucleic acid molecule of the ninth aspect of the present invention.

In another preferred embodiment, the expression vector is selected from the group consisting of: DNA, RNA, viral vectors, plasmids, transposons, other gene transfer systems, and combinations thereof. Preferably, the expression vector comprises a viral vector, such as lentivirus, adenovirus, AAV virus, retrovirus, or a combination thereof.

In another preferred embodiment, the expression vector further comprises elements selected from the group consisting of: promoters, WPRE transcriptional enhancer elements, long terminal repeat (LTR) sequences, etc.

In another preferred embodiment, the expression vector comprises one or more promoters operably linked to the polynucleotide or a fragment thereof, enhancer, intron, transcriptional termination signal, polyadenylation sequence, replication origin, selective marker, nucleic acid restriction site, and/or homologous recombination site.

In another preferred embodiment, the expression vector has a nucleotide sequence as set forth in SEQ ID NO: 3.

In another preferred embodiment, the nucleotide sequence of the expression vector has at least 50% identity, preferably has at least 60%, 70%, 80%, 90%, 95%, 99%, or 100% identity with the sequence as set forth in SEQ ID NO: 3.

In the eleventh aspect of the present invention, is provided a host cell comprising the expression vector of the tenth aspect of the present invention, or having the nucleic acid molecule of the ninth aspect of the present invention integrated into its genome.

In the twelfth aspect of the present invention, is provided a use of the nucleic acid molecule of the ninth aspect of the present invention, the expression vector of the tenth aspect of the present invention, or the host cell of the eleventh aspect of the present invention, in the manufacture of a formulation or composition for the treatment of hereditary angioedema.

In another preferred embodiment, the hereditary angioedema is of the C1-INH deficiency type.

In another preferred embodiment, the hereditary angioedema comprises HAE type I and HAE type II.

In the thirteenth aspect of the present invention, is provided a pharmaceutical composition, comprising:

(i) the nucleic acid molecule of the ninth aspect of the present invention, the expression vector of the tenth aspect of the present invention, or the host cell of the eleventh aspect of the present invention, as an active ingredient; and
(ii) a pharmaceutically acceptable carrier, diluent, or excipient.

In another preferred embodiment, component (i) accounts for 0.1-99.9 wt%, preferably 10-99.9 wt%, and more preferably 70-99 wt% of the total weight of the pharmaceutical composition.

In another preferred embodiment, the dosage form of the pharmaceutical composition is selected from the group consisting of: lyophilized dosage forms and liquid dosage forms.

In another preferred embodiment, the dosage form of the pharmaceutical composition is an injection.

In another preferred embodiment, the method of administration of the pharmaceutical composition includes but is not limited to, intravenous injection, oral administration, subcutaneous injection, intramuscular injection, etc.

In another preferred embodiment, the pharmaceutically acceptable carrier includes but is not limited to: solvents, dispersion media, coatings, antibacterial or antifungal agents, isotonic agents, and absorption-delaying agents, etc.

In another preferred embodiment, the pharmaceutically acceptable carrier is an injection carrier; and preferably, the pharmaceutically acceptable carrier comprises saline, wherein the saline includes but is not limited to: buffered saline, normal saline, phosphate buffer, citrate buffer, acetate buffer, bicarbonate buffer, sucrose solution, salt solution, polysorbate solution, or a combination thereof.

In another preferred embodiment, the pharmaceutically acceptable carrier may further comprise additives, including but not limited to: stabilizers, preservatives, transfection enhancers that facilitate cellular uptake, or combinations thereof.

In another preferred embodiment, the pharmaceutical composition may be used alone or in combination for the treatment of hereditary angioedema.

In another preferred embodiment, the combination use comprises: coadministration with other medicaments for treating hereditary angioedema.

In another preferred embodiment, the other medicaments for treating hereditary angioedema comprise: plasma-derived C1 esterase inhibitors, recombinant human C1 esterase inhibitors, bradykinin receptor antagonists, plasma kallikrein inhibitors, danazol, tranexamic acid, or combinations thereof.

In the fourteenth aspect of the present invention, is provided a method for treating hereditary angioedema, comprising a step of: administering the expression vector of the tenth aspect of the present invention or the pharmaceutical composition of the thirteenth aspect of the present invention, to a subject in need thereof.

In another preferred embodiment, the method of administration is intravenous injection.

In another preferred embodiment, the subject in need comprises a human and a non-human mammal.

In another preferred embodiment, the expression vector is an AAV vector.

In another preferred embodiment, the dosage of the AAV vector is 6E11 vg/kg to 6E13 vg/kg, preferably 2E12 vg/kg to 4E13 vg/kg, and more preferably 6E12 vg/kg to 2E13 vg/kg.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

## Description of the drawings

Figure 1 illustrates the results of detecting the expression of codon-optimized A, B, C genes and WT gene using Western blot method with FLAG antibody; wherein Fig. 1A illustrates the immunoblot image, and Fig. 1B illustrates the gray value ratio (relative protein content).

Figure 2 illustrates the results of detecting the expression of codon-optimized A, B, C genes and WT gene using Western blot method with SerpinG1 antibody; wherein Fig. 2A illustrates the immunoblot image, and Fig. 2B illustrates the gray value ratio (relative protein content).

Figure 3 illustrates the structure of the Y602 expression cassette.

Figure 4 illustrates the C1-INH content in the plasma of KO/KO mice and control mice at 1 week (left) and 2 weeks (right) after the administration of Y602.

Figure 5 illustrates the efficacy comparison data of Y602 and Y508 expression cassettes.

Figure 6 illustrates the structure of the GS 1196-016 expression cassette.

Figure 7 illustrates the C1-INH activity in cynomolgus monkeys 1 week after the injection of the GS1196-016-delivering AAV vector.

Figure 8 illustrates a comparison of C1-INH expression in mice mediated by the expression cassette of the present invention and other expression cassettes.

## Detailed description

After extensive and in-depth research, and through numerous screenings, the inventors have, for the first time, developed an expression cassette for target genes and established an AAV-based gene delivery system. By performing codon optimization on the serpinG1 gene, the inventors also obtained highly-expressing nucleic acid molecules, based on which expression vectors capable of further enhancing C1-INH protein expression both *in vitro* and *in vivo,* along with related host cells, and applications thereof were developed. Based on the above nucleic acid molecules, expression cassettes and gene delivery systems, etc., a therapeutic method with low side-effects and lifelong benefits from a single administration is constructed in the present invention. On this basis, the present invention has been completed.

## Terms

To facilitate a better understanding of the present disclosure, certain terms are defined first. As used in the present application, unless otherwise specified herein, each of the following terms shall have the meanings provided below.

As used herein, the term "about", when referring to a specifically enumerated value, means that the value may vary by up to 1% from the enumerated value. For example, as used herein, the expression "about 100" encompasses all values between 99 and 101 (e.g., 99.1, 99.2, 99.3, 99.4, etc.).

As used herein, the term "containing" or "comprising (including)" may be open-ended, semi-closed, or close-ended. In other words, the term also includes "essentially consisting of" or "consisting of".

As used herein, the term "treatment" refers to administering to a patient a therapeutic agent, which comprises the gene delivery system, expression vector or pharmaceutical composition of the present invention, for an internal or external use. The patient has one or more disease symptoms, and the therapeutic agent is known to be effective against these symptoms. Typically, the patient is given a therapeutic agent at a dose that is effective in alleviating the symptoms of one or more diseases (therapeutically effective dose).

As used herein, the term "optional" or "optionally" means that the event or situation described subsequently may occur but are not necessarily required to occur.

Sequence identity is determined by comparing two aligned sequences along a predefined comparison window (which may be 50%, 60%, 70%, 80%, 90%, 95%, or 100% of the length of the reference nucleotide sequence or protein), and calculating the number of positions where identical residues appear. This is typically expressed as a percentage. The measurement of sequence identity of nucleotide sequences is a well-known method among those skilled in the art.

As used herein, the term "subject in need" refers to any mammal or non-mammal. Mammals include but are not limited to humans, vertebrates such as rodents, non-human primates, cattle, horses, dogs, cats, pigs, sheep, and goats.

As used herein, the terms "AAV vector", "recombinant AAV vector", "recombinant adeno-associated virus vector", "rAAV" and "recombinant virus" may be used interchangeably, and they all refer to modified AAV viral particles used for delivering, transducing, and specifically expressing the contained exogenous target genes at the targeted site, and preferably the targeted site is the liver.

**Expression cassette**

Typically, the expression cassette of the present invention has a structure shown in formula I from the 5' to 3' end:

$$Z1\text{-}Z2\text{-}Z3\text{-}Z4\text{-}Z5\text{-}Z6\text{-}Z7\text{-}Z8 \quad (I)$$

wherein each "-" is independently a bond or a nucleotide linker sequence;
Z1 is an HCR element;
Z2 is a DSE element;
Z3 is a TPL element;
Z4 is an eMlp element;
Z5 is an intron element;
Z6 is absent or is a Kozak sequence;
Z7 is a target gene; and
Z8 is a poly(A) element.

It should be understood that the target gene in the expression cassette of the present invention, namely Z7, may be any optional target gene of interest. The expression cassette of the present invention enables the expression of any optional target gene.

Preferably, the expression cassette of the present invention is used for the expression of the serpinG1 gene, FIX gene, PAH (phenylketonuria), GBA1 gene (Gaucher's disease), GLA gene (Fabry disease), IDS (mucopolysaccharidosis type II), G6P (favism), GAA (Pompe disease), luciferase gene, CFTR gene (cystic fibrosis), LDLR gene (familial hypercholesterolemia), $\alpha$-globin gene, $\beta$-globin gene (thalassemia), APC gene (familial adenomatous polyposis), SLC26A4 gene, GJB2 gene (congenital deafness), TYR gene, OCA2 gene, TYRP1 gene, SLC45A2 gene (albinism), and a combination thereof.

The term "HCR element" used herein refers to the human apolipoprotein hepatic control region. In specific embodiments, the sequence of the HCR element used in the expression cassette of the present invention is selected from the group consisting of: SEQ ID NO: 4, SEQ ID NO: 10, and a combination thereof.

The term "DSE element" used herein refers to a chimeric promoter. In specific embodiments, the sequence of the DSE element used in the expression cassette of the present invention is as set forth in SEQ ID NO: 5.

The term "TPL element" used herein refers to the tripartite leader sequence of adenovirus. Based on the teachings of the present invention, those skilled in the art may use wild-type TPL or further optimize the wild-type TPL sequence to obtain functionally enhanced mutant or chimeric TPL sequences. For example, in specific embodiments, the TPL sequence used in the expression cassette of the present invention may be the wild-type TPL sequence SEQ ID NO: 6, or the chimeric TPL sequence SEQ ID NO: 11 composed of a wild-type TPL sequence and a segment of the $\beta$-globin intron. For chimeric TPL sequences, in addition to the $\beta$-globin intron and a segment thereof, other introns or segments thereof commonly used in the field (exemplary introns can be found in the term "intron") can also be combined with wild-type TPL sequences. The introns suitable for combining with wild-type TPL sequences to form chimeric TPL sequences may be different and complete introns or combinations thereof, combinations of different segments of different introns, or combinations of complete introns with segments of other introns.

The term "eMlp element" used herein refers to the enhancer element of the major late promoter of adenovirus. In specific embodiments, the sequence of the eMlp element used in the expression cassette of the present invention is as set forth in SEQ ID NO: 7.

The term "Kozak sequence" used herein refers to a nucleotide sequence located behind the 5' cap structure of eukaryotic mRNA, usually GCCACCAUGG (SEQ ID NO: 16), which can bind to translation initiation factors and mediate the translation initiation of mRNA containing the 5' cap structure. Based on the teachings of the present invention, a person skilled in the art can further optimize the Kozak sequence to enhance its function in the expression cassette of the present invention.

The term "intron" used herein bears a well-known meaning recognized by those skilled in the art. Those skilled in the art are aware of the various introns that can be utilized in the expression cassettes of the present invention. In specific embodiments, the introns of the present invention may be the β-globin intron, SV40 intron, HBB2 intron, VH4 intron, U12 intron, Chi intron, RHD intron, an intron of SRB gene, an intron of Minute Virus of Mice (MVM), etc. Exemplary intron sequences are provided in Lu et al., (2013) Molecular Therapy 21(5):954-63 and Lu et al., (2017) Human Gene Therapy 28(1):125-34, which are incorporated herein by reference in their entirety. Those skilled in the art can also comprehend that the introns applicable to the expression cassettes of the present invention may be combinations of various introns or segments thereof (i.e., chimeric introns). In specific embodiments, the introns applicable to the expression cassettes of the present invention may be combinations of different and complete introns, combinations of different segments of different introns, or combinations of complete introns with segments of other introns.

Typically, the expression cassette of the present invention is the expression cassette Y602 or expression cassette GS1196-016, with the nucleotide sequence as set forth in SEQ ID NO: 3 or SEQ ID NO: 15.

The advantage of the expression cassette of the present invention is that, on the one hand, compared with conventional expression cassettes in the prior art, the expression cassette of the present invention can achieve higher *in vitro* and/or *in vivo* expression levels; on the other hand, the inventors found through screening that compared with other expression cassettes constructed in the same batch by the inventors (such as the Y508 expression cassette), the expression cassette of the present invention has unexpectedly better effects.

**Nucleic acid molecule**

In the present invention, is provided a nucleic acid molecule encoding the expression cassette of the first aspect of the present invention. The nucleic acid molecule of the present invention may be in the form of a DNA or RNA. DNA forms include cDNA, genomic DNA, or artificially synthesized DNA. DNA may be single-stranded or double-stranded. DNA may be a coding chain or a noncoding chain.

In the present invention, is also provided the nucleic acid molecule of the ninth aspect of the present invention, the nucleotide sequence of which has at least 87% identity, preferably at least 88%, 89%, 90%, 91%, 92%, 93%, 94%, 95%, 96%, 97%, 98%, 99% or 100% identity to the sequence as set forth in SEQ ID NO: 1.

Once a relevant sequence is obtained, the recombination method can be used to obtain them in large quantities. This usually involves cloning it into a vector, then transferring it into a cell, and isolating the relevant sequence from the proliferating host cell using conventional methods. The biological molecules (nucleic acids, proteins, etc.) involved in the present invention include biological molecules that exist in isolated form.

**Expression vector**

The present invention also relates to vectors comprising appropriate nucleic acid molecules as described above and appropriate promoters or control sequences. These vectors can be used to transform appropriate host cells to enable them to express proteins.

**Host cell**

Host cells may be prokaryotic cells like bacterial cells; or lower eukaryotic cells like yeast cells; or higher eukaryotic cells like mammalian cells. Representative examples include: bacterial cells such as *Escherichia coli, Streptomyces, Salmonella typhimurium;* fungal cells such as yeast cells; insect cells such as *Drosophila* S2 or Sf9; animal cells such as CHO, COS7, 293 cells, etc. The transformation of host cells with recombinant nucleic acid molecules can be carried out using conventional techniques well known to those skilled in the art.

**Formulation and composition (pharmaceutical composition)**

In the present invention, is provided a formulation or composition (preferably a pharmaceutical composition) comprising (a) the expression cassette of the first aspect of the present invention, the nucleic acid molecule of the second aspect of the present invention, the expression vector of the third aspect of the present invention, the host cell of the fourth aspect of the present invention, or the gene delivery system of the fifth aspect of the present invention, as an active ingredient; and (b) a pharmaceutically acceptable carrier, diluent, or excipient.

In the present invention, is also provided a formulation or composition (preferably a pharmaceutical composition) comprising (a) the nucleic acid molecule of the ninth aspect of the present invention, the expression vector of the tenth aspect of the present invention, or the host cell of the eleventh aspect of the present invention, as an active ingredient; and (b) a pharmaceutically acceptable carrier, diluent, or excipient.

Typically, the formulation or composition of the present invention is used for gene therapy, preferably for the treatment of hereditary angioedema or hemophilia.

For the convenience of clinical application, the formulation or composition of the present invention may be included in an injection medicament delivery device (such as an injection needle), which may comprise a single dose of the pharmaceutical composition. The injection medicament delivery device can be included in a medication box for convenient storage and use. During transportation, small containers containing medicament suspensions need to be placed in dry ice. Normally, they should be stored in a -80 °C refrigerator.

The formulation or composition of the present invention may also include an instruction manual to facilitate those skilled in the art to use it in a correct manner.

The formulation or composition of the present invention may be administered in a safe and effective dose, wherein "safe and effective dose" refers to an amount of active ingredient sufficient to significantly alleviate the condition or symptoms without causing serious side-effects.

The safe and effective dose of the present invention may vary depending on the mode of administration and the severity of the disease to be treated. The selection of the preferred safe and effective dose can be determined by ordinary technical personnel in this field based on various factors (such as through clinical trials). The factors mentioned include but are not limited to: pharmacokinetic parameters of the medicament, such as medicament tissue distribution, bioavailability, metabolism, half-life, etc.; the severity of the disease to be treated, the patient's weight, the patient's immune status, the route of administration, etc. For example, depending on the urgency of the therapeutic condition, the dose may be administered in divided daily doses or be proportionally reduced.

"Pharmaceutically acceptable carrier, diluent, or excipient" refer to: one or more compatible solid or liquid fillers or gel substances that are suitable for human use and must have sufficient purity and sufficiently low toxicity. "Compatibility" as used herein means that the components in the formulation or composition can be mixed with the active ingredients of the present invention without significantly reducing the efficacy of the active ingredients.

The formulation or composition can be liquid or solid, such as powder, gel or paste. Preferably, the composition is a liquid, and more preferably an injectable liquid. Suitable excipients are known to those skilled in the art.

The formulation or composition may contain physiologically acceptable sterile aqueous or non-aqueous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and their suitable mixtures.

Some examples of pharmaceutically acceptable carriers are cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agents (such as sodium lauryl sulfate), coloring agents, flavoring agents, stabilizers, antioxidants, preservatives, pyrogen-free water, etc.

**Hereditary angioedema**

Hereditary angioedema (HAE), also known as C1 inhibitor deficiency, is characterized by a deficiency in the quantity or functional defect of C1 inhibitor. It is an autosomal dominant genetic disease with an incidence rate of 1/10,000 - 1/50,000. Clinically, it presents as recurrent and unpredictable subcutaneous and submucosal edema. If laryngeal edema occurs, it can endanger life due to asphyxiation. Currently, hereditary angioedema cannot be cured, but its attacks can be prevented and the symptoms during acute episodes can be controlled through methods such as medication.

However, the treatment with small-molecule medicaments requires weekly or even daily administration and is associated with numerous side-effects. When using inhibitors for treatment, the medicaments need to be administered during the onset period, which poses a risk of delayed treatment and the problem of poor patient compliance.

**Treatment for hereditary angioedema**

In conventional treatments, therapies for hereditary angioedema are categorized into general treatment, acute-phase treatment, and prophylactic treatment. Prophylactic treatment is further divided into short-term treatment and long-term sustained treatment based on the treatment cycle.

The above conventional treatments typically utilize plasma-derived C1 esterase inhibitors, recombinant human C1 esterase inhibitors, bradykinin receptor antagonists, plasma kallikrein inhibitors, danazol and other small-molecule medicaments or formulations. These treatments can achieve effects, such as alleviating acute episodes and providing short- or long-term prevention, to a certain extent, but remain associated with challenges, such as numerous side-effects, frequent administration, delayed treatment, and poor patient adherence.

Specifically, for example, current first-line therapies for hereditary angioedema include small-molecule medicaments and C1/Kallikrein inhibitors, with treatment strategies focusing on on-demand and prophylactic regimens. However, small-molecule medicaments require weekly or even daily administration and are accompanied by numerous side-effects, while inhibitor therapies must be administered during acute episodes, posing risks of delayed treatment and poor patient compliance.

The present invention introduces a novel treatment with low side-effects and the potential to provide lifelong benefits from a single administration. Typically, the present invention employs an AAV-based gene delivery system to specifically express the expression cassette of the present invention, wherein the target gene in the expression cassette is the serpinG1 gene. This expression cassette and gene delivery system enable high-level expression of C1-INH protein in a subject in need thereof, thereby effectively treating hereditary angioedema.

## C1-INH

Hereditary angioedema (HAE) can be classified into C1-INH deficiency-type and non-C1-INH deficiency-type.

The C1-INH deficiency-type is caused by mutations in the C1-INH gene, clinically categorized into Type 1 and Type 2 based on laboratory C1-INH concentration measurements.

Type 1 HAE: Characterized by reduced C4 concentration and decreased C1-INH concentration in laboratory tests. This type accounts for the majority of HAE cases in China.

Type 2 HAE: Characterized by reduced C4 concentration, but normal or elevated C1-INH concentration with impaired C1-INH function.

The non-C1-INH deficiency-type is associated with mutations in genes such as F12, ANGPTI, and PLG.

In the present invention, the nucleic acid molecules, expression cassettes, gene delivery systems, formulations, or compositions of the present invention primarily target C1-INH deficiency-type HAE (including Type 1 HAE and Type 2 HAE), and they can produce therapeutic effects on both reduced C1-INH concentration and impaired C1-INH function in patients.

## SerpinG1 gene

The serpinG1 gene is the coding gene for the C1-INH protein. In the present invention, wild-type serpinG1 genes were codon-optimized to generate serping1 optiA, serping1 optiB, and serping1 optiC genes, abbreviated as Gene A, Gene B, and Gene C.

Preferably, the nucleic acid molecule of the present invention is the Gene B as set forth in SEQ ID NO: 1, or a nucleic acid molecule having at least 87% sequence identity, preferably at least 88%, 89%, 90%, 91%, 92%, 93%, 94%, 95%, 96%, 97%, 98%, 99%, or 100% identity to the sequence of SEQ ID NO: 1.

## Hemophilia and the FIX gene

Hemophilia is an X-chromosome-linked recessive hereditary hemorrhagic disease. Clinically, it is divided into two types: hemophilia A (coagulation factor VIII-deficiency) and hemophilia B (coagulation factor IX-deficiency), which are caused by gene mutations in coagulation factor VIII (FVIII) and coagulation factor IX (FIX) respectively. In the male population, the incidence of hemophilia A is approximately 1 in 5,000, and that of hemophilia B is about 1 in 25,000. Among all male hemophilia patients, hemophilia A accounts for about 80% - 85%, while hemophilia B accounts for about 15% - 20%. Female hemophilia patients are extremely rare.

Coagulation factor IX (FIX) is an important coagulation factor in the coagulation process. It is activated by tissue factor and activated coagulation factor VII (TF/FVIIa) in the initial stage of coagulation. The generated activated FIX quickly forms a tetrameric complex with TF/FVIIa and tissue factor pathway inhibitor (TFPI), thereby terminating the activation of coagulation factor X (FX) and FIX mediated by TF/FVIIa. However, with the assistance of activated coagulation factor VIII (FVIIIa), FXa generated by the activation of activated FIX (FIXa) is crucial for maintaining the coagulation process and ultimately achieving hemostasis; thus, it plays an irreplaceable role in the hemostasis process. The gene encoding FIX (F9) is located on the long arm of the X-chromosome (Xq27.1 - q27.2) and consists of 8 exons and 7 introns. Mutations in this gene are the fundamental cause of hemophilia B.

**Adeno-associated virus**

Currently, numerous viral vectors have been employed in gene therapy, including adenoviruses, retroviruses, lentiviruses, and adeno-associated viruses (AAV). Among these, AAV, characterized by its low immunogenicity, has gained widespread recognition.

Adeno-associated virus, also known as adeno-dependent virus, belongs to the *Dependoparvovirus* genus of the *Parvoviridae* family. It is the simplest single-stranded DNA defective virus identified to date, requiring an auxiliary virus (typically adenovirus) for replication. AAV can infect a variety of cells while stably integrating into the genome of infected cells in a site-specific manner. They can infect a large range of cells without affecting cell growth, morphology, or differentiation, and they appear unrelated to human pathology.

AAV demonstrates notable safety, high infection efficiency, and the capacity to mediate long-term gene expression. Despite its low immunogenicity, it is often reported in clinical practice that systemic administration of AAV can trigger significant immune responses, mainly due to the use of high doses.

The expression cassette provided in the present invention, through its innovative design, enhances the expression level of the target gene. Consequently, constructing an AAV vector comprising the expression cassette of the present invention can reduce the required dosage of the AAV vector, thereby preserving the advantages of long-term expression and localized delivery of the AAV vector, while minimizing adverse effects associated with high-dose administration of the AAV vector.

**Gene delivery system**

In the present invention, an AAV-based gene delivery system was established, wherein AAV refers to a genetically engineered recombinant adeno-associated virus vector. AAV contains an inverted terminal repeat (ITR) region of approximately 145 base pairs at each end. This region serves as the origin of viral replication and plays a decisive role in viral packaging. The rest of the genome is divided into two important regions with encapsidation functions: the left part of the genome containing the rep gene involved in viral replication and viral gene expression; and the right part of the genome containing the cap gene encoding the viral capsid protein. Recombinant adeno-associated virus vectors (rAAVs) are derived from non-pathogenic wild-type adeno-associated viruses. Due to their advantages such as good safety, wide range of host cells (both dividing and non-dividing cells), low immunogenicity, and long-term expression of exogenous genes *in vivo,* they are regarded as one of the most promising gene transfer vectors and are widely used in gene therapy and vaccine research worldwide. After more than a decade of research, the biological characteristics of recombinant adeno-associated viruses have been thoroughly understood, and a large amount of data has been accumulated, especially regarding their application effects in various cells, tissues, and *in vivo* experiments. In medical research, rAAV is used in gene therapy research for various diseases (including *in vivo* and *in vitro* experiments). Meanwhile, as a unique gene transfer vector, it is also widely used in gene function research, disease model construction, and preparation of gene-knockout mice.

The AAV capsid protein determines the tissue-cell specificity of AAV. In the present invention, there are no particular restrictions on the applicable AAV capsid proteins. The AAV capsid protein can be a natural AAV capsid protein or a genetically engineered AAV capsid protein. The AAV capsid protein may include a capsid protein of the AAV selected from the group consisting of: AAV1, AAV2, AAV3, AAV4, AAV5, AAV6, AAV7, AAV8, AAV9, AAV10, AAV11, AAV12, and AAV-DJ. Preferably, the AAV capsid protein of the present invention is the capsid protein of AAV8, which may have the amino acid sequence as set forth in SEQ ID NO: 9.

AAV vectors can be prepared using standard methods in the art. Any serotype of adeno-associated virus is suitable. Methods for purifying vectors can be found in, for example, U.S. Patent Nos. 6,566,118, 6,989,264, and 6,995,006, the disclosures of which are incorporated herein by reference in their entirety. The preparation of hybrid vectors is described in, for example, PCT Application No. PCT/US2005/027091, the disclosure of which is incorporated herein by reference in its entirety. The use of AAV-derived vectors for *in vitro* and *in vivo* gene transfer has been described (see, for example, International Patent Application Publication Nos. WO91/18088 and WO93/09239; U.S. Patent Nos. 4,797,368, 6,596,535, and 5,139,941, and European Patent No. 0488528, all of which are incorporated herein by reference in their entirety). These patent publications describe various AAV-derived constructs in which the rep and/or cap genes are deleted and replaced with the gene of interest, as well as the use of these constructs for transferring the gene of interest *in vitro* (into cultured cells) or *in vivo* (directly into organisms).

In a specific embodiment, the production of AAV vectors requires a DNA plasmid containing ITR-L, the recombinant target genome, and ITR-R, wherein ITR-L and ITR-R are located on either side of the recombinant genome. The above-mentioned DNA plasmid, the plasmid encoding the AAV cap/rep genes, and the helper genes provided by adenovirus or herpes virus can be introduced into a suitable host cell simultaneously using known techniques, such as transfection, to produce AAV viral vectors. The DNA plasmid can be expressed in the host cell and packaged into viral particles.

**Therapeutic method**

The therapeutic methods of the present invention typically employ various administration routes, to administer various expression vectors (e.g., DNA, RNA, viral vectors, plasmids, transposons, other gene transfer systems, or combinations thereof), host cells, formulations, or compositions comprising the expression cassette of the present invention, to a subject in need thereof.

For example, the gene therapy method of the eighth aspect of the present invention, or the method for treating hereditary angioedema of the fourteenth aspect of the present invention is provided in the present invention.

Typically, the present invention utilizes AAV-based gene delivery systems (i.e., AAV vectors) to specifically express the expression cassette of the present invention. On the one hand, these therapeutic methods enhance the expression level of the target gene (protein) through the specialized design of the expression cassette, thereby reducing the required dosage of the corresponding expression vector and minimizing adverse effects; on the other hand, gene delivery systems administered via local routes can effectively avoid adverse reactions caused by systemic administration.

The therapeutic methods of the present invention generally fall under gene therapy, wherein target genes for gene therapy are categorized into three major types: normal genes, antisense genes, and suicide genes.

Normal genes are isolated from healthy individuals. They can replace defective genes via homologous recombination or compensate for their physiological functions through their expressed products. These genes are commonly used to treat various genetic disorders caused by gene defects, such as hemophilia, thalassemia, and hereditary angioedema.

Antisense genes are primarily used to treat acquired molecular diseases. Their therapeutic mechanism involves the *in vivo* expression products (RNA) of the antisense genes, which are either complementary to the genes encoding viral activators or complementary to tumor gene mRNA, to block the expression of the virus gene or tumor gene. Examples of antisense genes include the antisense BL-2 oligonucleotides, antisense MYC fragments, and antisense RNA of HIV-1 genome gene fragments (such as pol gene, env gene, vif gene), etc.

Suicide genes are a class of genes encoding enzymes that kill cancerous cells. These genes are found in viruses, bacteria, and fungi, and they can convert harmless cellular metabolic products into toxic chemicals. Examples of suicide genes include the HSV-tk gene, CD gene, or suicide gene prodrug systems such as the gpt-6-TX system and P450 2BI-CPA system, etc.

In addition, the main gene therapy methods for tumors include: tumor suppressor gene therapy (antisense BL-2 oligonucleotides, antisense MYC fragments), gene-modified tumor vaccine method, immune gene therapy (tumor cytokine gene therapy, tumor MHC gene therapy, tumor antigen-targeted gene therapy (such as TSA gene or TAA gene), tumor costimulatory molecule gene therapy (such as B7-1 (CD80), B7-2 (CD86), ICAM-1 (CD54)), etc.) and suicide gene therapy.

All types of target genes used for various therapeutic purposes and treatment methods are included in the scope of the present invention.

**Main advantages of the present invention include:**

1. The expression cassette of the present invention, through its innovative design, enhances the expression of the target gene both *in vitro* and *in vivo*. This improvement reduces economic and time costs in industrial production. Furthermore, the high-level expression of the target protein enables a reduction in the dosage of related medicaments comprising the expression cassette of the present invention, thereby minimizing the occurrence of adverse effects.

2. The treatment method provided in the present invention can reduce side effects through local administration and relatively low medication dosage on the one hand, and achieve lifelong benefits with long-term expression ability after a single administration on the other hand.

3. The nucleic acid molecule of the present invention can increase the expression level of serpinG1 gene, which is beneficial for reducing production costs, as well as lowering the dosage of medicaments comprising the nucleic acid molecule of the present invention.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

**Sequence information**

**serpinG1 codon-optimized sequence (SEQ ID NO: 1)**

```
ATGGCTTCTAGGCTGACACTGCTGACACTGCTGCTGCTGCTGCTGGCTGGAGATAGAGCCTCCAGCAATCCCAATGCCACA
TCCAGCTCCAGCCAAGACCCTGAGAGCCTCCAAGACAGAGGTGAGGGCAAAGTGGCCACAACTGTGATCTCCAAGATGCT
CTTTGTGGAGCCTATTCTGGAGGTGAGCTCTCTGCCCACCACCAATTCCACAACCAATTCTGCCACCAAGATCACTGCCAA
CACCACTGATGAGCCTACAACACAGCCTACAACAGAACCTACCACACAGCCCACCATTCAGCCCACCCAGCCCACCCACAC
AGCTGCCTACTGACAGCCCTACCCAGCCTACCACTGGCAGCTTCTGTCCTGGACCTGTGACACTGTGCTCTGATCTGGAGA
GCCATAGCACAGAGGCTGTGCTGGGTGATGCTCTGGTGGATTTCTCTCTGAAGCTGTATCATGCCTTCTCTGCCATGAAGA
AGGTAGAGACAAACATGGCCTTTAGCCCCTTCTCCATTGCTTCTCTGCTCACACAAGTGCTGCTGGGTGCTGGTGAGAATA
CCAAGACCAATCTGGAGTCCATCCTCTCCTACCCCAAGGACTTTACATGTGTGCACCAAGCCCTCAAGGGATTCACCACCA
AGGGAGTGACAAGTGTGAGCCAAATCTTCCACTCCCCTGACCTGGCCATCAGAGACACCTTTGTGAATGCCTCTAGGACAC
TGTACAGCTCCTCCCCCAGAGTGCTGAGCAATAATAGTGATGCCAATCTGGAGCTCATCAATACATGGGTGGCTAAGAATA
CCAACAACAAGATCTCTAGGCTGCTGGATTCCCTCCCTTCTGACACAAGACTGGTGCTGCTGAATGCCATTTATCTGAGTG
CCAAGTGGAAGACCACATTTGACCCCAAGAAGACAAGAATGGAGCCCTTTCACTTCAAGAACTCTGTCATCAAGGTGCCC
ATGATGAACAGTAAGAAGTATCCTGTGGCCCACTTCATTGACCAGACCCTCAAGGCCAAAGTGGGACAGCTGCAGCTGAG
CCACAATCGTCTCTGGTGATTCTGGTGCCCCAGAATCTGAAACATAGACTGGAGGACATGGAACAAGCTCTGAGCCCCA
GTGTCTTTAAGGCCATCATGGAGAAGCTGGAGATGTCCAAGTTCCAGCCCACACTCCTCACACTGCCTAGAATCAAAGTCA
CCACAAGCCAAGACATGCTGAGCATCATGGAAAAGCTGGAATTCTTTGACTTCAGCTATGATCTGAACCTCTGTGGACTCA
CAGAGGATCCTGATCTGCAAGTGAGTGCCATGCAGCACCAGACTGTGCTGGAACTGACTGAAACTGGAGTGGAGGCTGCT
GCTGCCAGTGCTATCAGTGTGGCTAGAACACTGCTGGTGTTTGAGGTGCAGCAGCCCTTTCTGTTTGTCCTCTGGGATCAGC
AGCATAAGTTCCCTGTGTTCATGGGAAGGGTGTATGATCCTAGAGCCTGA
```

serpinG1 **wild-type sequence (SEQ** ID NO: **2)**

```
ATGGCCTCCAGGCTGACCCTGCTGACCCTCCTGCTGCTGCTGCTGGCTGGGGATAGAGCCTCCTCAAATCCAAATGCTACC
AGCTCCAGCTCCCAGGATCCAGAGAGTTTGCAAGACAGAGGCGAAGGGAAGGTCGCAACAACAGTTATCTCCAAGATGCT
ATTCGTTGAACCCATCCTGGAGGTTTCCAGCTTGCCGACAACCAACTCAACAACCAATTCAGCCACCAAAATAACAGCTAA
TACCACTGATGAACCCACCACACAACCCACCACAGAGCCCACCACCCAACCCACCATCCAACCCACCCAACCAACTACCC
AGCTCCCAACAGATTCTCCTACCCAGCCCACTACTGGGTCCTTCTGCCCAGGACCTGTTACTCTCTGCTCTGACTTGGAGAG
TCATTCAACAGAGGCCGTGTTGGGGGGATGCTTTGGTAGATTTCTCCCTGAAGCTCTACCACGCCTTCTCAGCAATGAAGAA
GGTGGAGACCAACATGGCCTTTTCCCATTCAGCATCGCCAGCCTCCTTACCCAGGTCCTGCTCGGGGCTGGGGAGAACAC
CAAAACAAACCTGGAGAGCATCCTCTCTTACCCCAAGGACTTCACCTGTGTCCACCAGGCCCTGAAGGGCTTCACGACCAA
AGGTGTCACCTCAGTCTCTCAGATCTTCCACAGCCCAGACCTGGCCATAAGGGACACCTTTGTGAATGCCTCTCGGACCCT
GTACAGCAGCAGCCCCAGAGTCCTAAGCAACAACAGTGACGCCAACTTGGAGCTCATCAACACCTGGGTGGCCAAGAACA
CCAACAACAAGATCAGCCGGCTGCTAGACAGTCTGCCCTCCGATACCCGCCTTGTCCTCCTCAATGCTATCTACCTGAGTG
CCAAGTGGAAGACAACATTTGATCCCAAGAAAACCAGAATGGAACCCTTTCACTTCAAAAACTCAGTTATAAAAGTGCCC
ATGATGAATAGCAAGAAGTACCCTGTGGCCCATTTCATTGACCAAACTTTGAAAGCCAAGGTGGGGCAGCTGCAGCTCTC
CCACAATCTGAGTTTGGTGATCCTGGTACCCCAGAACCTGAAACATCGTCTTGAAGACATGGAACAGGCTCTCAGCCCTTC
TGTTTTCAAGGCCATCATGGAGAAACTGGAGATGTCCAAGTTCCAGCCCACTCTCCTAACACTACCCCGCATCAAAGTGAC
GACCAGCCAGGATATGCTCTCAATCATGGAGAAATTGGAATTCTTCGATTTTTCTTATGACCTTAACCTGTGTGGGCTGAC
AGAGGACCCAGATCTTCAGGTTTCTGCGATGCAGCACCAGACAGTGCTGGAACTGACAGAGACTGGGGTGGAGGCGGCTG
CAGCCTCCGCCATCTCTGTGGCCCGCACCCTGCTGGTCTTTGAAGTGCAGCAGCCCTTCCTCTTCGTGCTCTGGGACCAGCA
GCACAAGTTCCCTGTCTTCATGGGGCGAGTATATGACCCCAGGGCCTAA
```

**Y602 expression cassette sequence (SEQ ID NO: 3)**

TAAAATGGGCAAACATGCTGTTTACTGAGCTGGGCACCGTAAAATGGGCAAACATTGCAAGCAGCAAACAGCAAACACAC
AGCCCTCCCTGCCTGCTGACCTTGGAGCTGGGGCAGAGGTCAGAGACCTCTCTGGGCCCATGCCACCTCCAACATCCACTC
GACCCCTTGGAATTTCGGTGGAGAGGAGCAGAGGTTGTCCTGGCGTGGTTTAGGTAGTGTGAGAGGGAGGACGCCGCTGT
TTACTGAGCTGGGCACAATGACCTTTGGCGAGCTGGACAGAGGGCCGGGCGCAGACGGGCAGGCGGGTGGGCAGCTCGG
CGCTGACCTTTGCCCTTAGTCCCTGTTTGCTCCTCCGATAACCGGGGTGACTTTGGTTAATCATTAACCAGCAACCACCCC
GTCGTCCCTGCGGGTCCACAGCTTAAATACGAACTCGAACGGGGCTCTGTCTCCTTAGCCAGGCACCACCACTGACCTGGG
ACAGTGAATCGGGCTCGCGGTTGAGGACAAACTCTTCGCGGTCTTTCCAGTACTCTTGGATCGGAAACCCGTCGGCCTCCG
AACGGTACTCCGCCACCGAGGGACCTGAGCGAGTCCGCATCGACCGGATCGGAAAACCTCTCGAGAAAGGCGTCTAACCA
GTCACAGTCGCAAGGTAGGCTGAGCACCGTGGCGGGCGGCAGCGGGTGGCGGTCGGGGTTGTTTCTGGCGGAGGTGCTGC
TGATGATGTAATTAAAGTAGGCGGTCTTGAGACGGCGGATGGTCGAGGTGAGGTGTGGCAGGCTTGAGATCCAGCTGTTG
GGGTGAGTACTCCCTCTCAAAAGCGGGCATTACTTCTGCGCTAAGATTGTCAGTTTCCAAAAACGAGGAGGATTTGATATT
CACCTGGCCCGATCTGGCCATACACTTGAGTGACAATGACATCCACTTTGCCTTTCTCTCCACAGGTGTCCACTCCCAGGTC
CAAGTTTAAACTCTCTAAGGTAAATATAAAATTTTTAAGTGTATAATGTGTTAAACTACTGATTCTAATTGTTTCTCTCTTTT
AGATTCCAACCTTTGGAACTGAGCCACCATGGCTTCTAGGCTGACACTGCTGACACTGCTGCTGCTGCTGCTGGCTGGAGA
TAGAGCCTCCAGCAATCCCAATGCCACATCCAGCTCCAGCCAAGACCCTGAGAGCCTCCAAGACAGAGGTGAGGGCAAAG
TGGCCACAACTGTGATCTCCAAGATGCTCTTTGTGGAGCCTATTCTGGAGGTGAGCTCTCTGCCCACCACCAATTCCACAA
CCAATTCTGCCACCAAGATCACTGCCAACACCACTGATGAGCCTACAACACAGCCTACAACAGAACCTACCACACAGCCC
ACCATTCAGCCCACCCAGCCCACCACACAGCTGCCTACTGACAGCCCTACCCAGCCTACCACTGGCAGCTTCTGTCCTGGA
CCTGTGACACTGTGCTCTGATCTGGAGAGCCATAGCACAGAGGCTGTGCTGGGTGATGCTCTGGTGGATTTCTCTCTGAAG
CTGTATCATGCCTTCTCTGCCATGAAGAAGGTAGAGACAAACATGGCCTTTAGCCCCTTCTCCATTGCTTCTCTGCTCACAC
AAGTGCTGCTGGGTGCTGGTGAGAATACCAAGACCAATCTGGAGTCCATCCTCTCCTACCCCAAGGACTTTACATGTGTGC
ACCAAGCCCTCAAGGGATTCACCACCAAGGGAGTGACAAGTGTGAGCCAAATCTTCCACTCCCTGACCTGGCCATCAGA
GACACCTTTGTGAATGCCTCTAGGACACTGTACAGCTCCTCCCCCAGAGTGCTGAGCAATAATAGTGATGCCAATCTGGAG
CTCATCAATACATGGGTGGCTAAGAATACCAACAACAAGATCTCTAGGCTGCTGGATTCCCTCCCTTCTGACACAAGACTG
GTGCTGCTGAATGCCATTTATCTGAGTGCCAAGTGGAAGACCACATTTGACCCCAAGAAGACAAGAATGGAGCCCTTTCA
CTTCAAGAACTCTGTCATCAAGGTGCCCATGATGAACAGTAAGAAGTATCCTGTGGCCCACTTCATTGACCAGACCCTCAA
GGCCAAAGTGGGACAGCTGCAGCTGAGCCACAATCTGTCTCTGGTGATTCTGGTGCCCCAGAATCTGAAACATAGACTGG
AGGACATGGAACAAGCTCTGAGCCCCAGTGTCTTTAAGGCCATCATGGAGAAGCTGGAGATGTCCAAGTTCCAGCCCACA
CTCCTCACACTGCCTAGAATCAAAGTCACCACAAGCCAAGACATGCTGAGCATCATGGAAAAGCTGGAATTCTTTGACTTC
AGCTATGATCTGAACCTCTGTGGACTCACAGAGGATCCTGATCTGCAAGTGAGTGCCATGCAGCACCAGACTGTGCTGGA
ACTGACTGAAACTGGAGTGGAGGCTGCTGCTGCCAGTGCTATCAGTGTGGCTAGAACACTGCTGGTGTTTGAGGTGCAGC
AGCCCTTTCTGTTTGTCCTCTGGGATCAGCAGCATAAGTTCCCTGTGTTCATGGGAAGGGTGTATGATCCTAGAGCCTGATT
GCATGTTAATCAATAAACCGGTTGATTCGTTTCAGTTGAACTTTGGTCTCCTGTGCTTATCTTATCGGTTTCCATAGCAACT
GGTTACACATTA

**HCR sequence (SEQ ID NO: 4)**

CCGTAAAATGGGCAAACATTGCAAGCAGCAAACAGCAAACACACAGCCCTCCCTGCCTGCTGACCTTGGAGCTGGGGCAG
AGGTCAGAGACCTCTCTGGGCCCATGCCACCTCCAACATCCACTCGACCCCTTGGAATTTCGGTGGAGAGGAGCAGAGGTT
GTCCTGGCGTGGTTTAGGTAGTGTGAGAGGG

**DSE sequence (SEQ ID NO: 5)**

AGGACGCCGCTGTTTACTGAGCTGGGCACAATGACCTTTGGCGAGCTGGACAGAGGGCCGGGCGCAGACGGGCAGGCGG
GTGGGCAGCTCGGCGCTGACCTTTGCCCTTAGTCCCTGTTTGCTCCTCCGATAACCGGGGTGACTTTGGTTAATCATTAACC
AGCAACCACCCCGTCGTCCCTGCGGGTCCACAGCTTAAATACGAACTCGAACGGGGCTCTGTCTCCTTAGC

**TPL sequence (SEQ ID NO: 6)**

GGGCTCGCGGTTGAGGACAAACTCTTCGCGGTCTTTCCAGTACTCTTGGATCGGAAACCCGTCGGCCTCCGAACGGTACTC
CGCCACCGAGGGACCTGAGCGAGTCCGCATCGACCGGATCGGAAAACCTCTCGAGAAAGGCGTCTAACCAGTCACAGTCG
CA

**eMLP sequence SEQ ID NO: 7)**

AGGTAGGCTGAGCACCGTGGCGGGCGGCAGCGGGTGGCGGTCGGGGTTGTTTCTGGCGGAGGTGCTGCTGATGATGTAAT
TAAAGTAGGCGGTCTTGAGACGGCGGATGGTCGAGGTGAGGTGTGGCAGGCTTGAGATCCAGCTGTTGGGGGTGAGTACTC
CCTCTCAAAAGCGGGCATTACTTCTGCGCTAAGATTGTCAGTTTCCAAAAACGAGGAGGATTTGATATTCACCTGGCCCGA
TCTGGCCATACACTTGAGTGACAATGACATCCACTTTGCCTTTCTCTCCACAGGTGTCCACTCCCAGGTCCAAGTTTAAACT

SV40 intron sequence (SEQ ID NO: 8)

CTCTAAGGTAAATATAAAATTTTTAAGTGTATAATGTGTTAAACTACTGATTCTAATTGTTTCTCTCTTTTAGATTCCAACCT
TTGGAACTGA

AAV8 cap sequence (SEQ ID NO: 9)

MAADGYLPDWLEDNLSEGIREWWALKPGAPKPKANQQKQDDGRGLVLPGYKYLGPFNGLDKGEPVNAADAAALEHDKAYD
QQLQAGDNPYLRYNHADAEFQERLQEDTSFGGNLGRAVFQAKKRVLEPLGLVEEGAKTAPGKKRPVEPSPQRSPDSSTGIGKK

GQQPARKRLNFGQTGDSESVPDPQPLGEPPAAPSGVGPNTMAAGGGAPMADNNEGADGVGSSSGNWHCDSTWLGDRVITTST
RTWALPTYNNHLYKQISNGTSGGATNDNTYFGYSTPWGYFDFNRFHCHFSPRDWQRLINNNWGFRPKRLSFKLFNIQVKEVTQ
NEGTKTIANNLTSTIQVFTDSEYQLPYVLGSAHQGCLPPFPADVFMIPQYGYLTLNNGSQAVGRSSFYCLEYFPSQMLRTGNNFQ
FTYTFEDVPFHSSYAHSQSLDRLMNPLIDQYLYYLSRTQTTGGTANTQTLGFSQGGPNTMANQAKNWLPGPCYRQQRVSTTTG
QNNNSNFAWTAGTKYHLNGRNSLANPGIAMATHKDDEERFFPSNGILIFGKQNAARDNADYSDVMLTSEEEIKTTNPVATEEY
GIVADNLQQQNTAPQIGTVNSQGALPGMVWQNRDVYLQGPIWAKIPHTDGNFHPSPLMGGFGLKHPPPQILIKNTPVPADPPTT
FNQSKLNSFITQYSTGQVSVEIEWELQKENSKRWNPEIQYTSNYYKSTSVDFAVNTEGVYSEPRPIGTRYLTRNL*

HCR2 sequence (SEQ ID NO: 10)

AGGCTCAGAGGCACACAGGAGTTTCTGGGCTCACCCTGCCCCCTTCCAACCCCTCAGTTCCCATCCTCCAGCAGCTGTTTGT
GTGCTGCCTCTGAAGTCCACACTGAACAAACTTCAGCCTACTCATGTCCCTAAAATGGGCAAACATTGCAAGCAGCAAAC
AGCAAACACACAGCCCTCCCTGCCTGCTGACCTTGGAGCTGGGGCAGAGGTCAGAGACCTCTCTGGGCCCATGCCACCTCC
AACATCCACTCGACCCCTTGGAATTTCGGTGGAGAGGAGCAGAGGTTGTCCTGGCGTGGTTTAGGTAGTGTGAGAGGG

TPL2 sequence (SEQ ID NO: 11)

GTGAGTCTATGGGACCCTTGATGTTTTCTTTCCCCTTCTTTTCTATGGTTAAGTTCATGTCATAGGAAGGGGAGAAGTAACA
GGGTACAGGGCTCGCGGTTGAGGACAAACTCTTCGCGGTCTTTCCAGTACTCTTGGATCGGAAACCCGTCGGCCTCCGAAC
GGTACTCCGCCACCGAGGGACCTGAGCGAGTCCGCATCGACCGGATCGGAAAACCTCTCGAGAAAGGCGTCTAACCAGTC
ACAGTCGCA

HBB2 intron segment sequence (SEQ ID NO: 12)

GTTCGGCTTCTGGCGTGTGACCGGCGGCTCTAGAGCCTCTGCTAACCATGTTCATGCCTTCTTCTTTTTCCTACAGCTCCTG
GGCAACGTGCTGGTTATTGTGCTGTCTCATCATTTTGGCA

PA3 poly(A) sequence (SEQ ID NO: 13)

TTGCATGTTAATCAATAAACCGGTTGATTCGTTTCAGTTGAACTTTGGTCTCCTGTGCTTATCTTATCGGTTTCCATAGCAAC
TGGTTACACATTA

BGH poly(A) sequence SEQ ID NO: 14)

CTGTGCCTTCTAGTTGCCAGCCATCTGTTGTTTGCCCCTCCCCCGTGCCTTCCTTGACCCTGGAAGGTGCCACTCCCACTGTC
CTTTCCTAATAAAATGAGGAAATTGCATCGCATTGTCTGAGTAGGTGTCATTCTATTCTGGGGGGTGGGGTGGGGCAGGAC
AGCAAGGGGGAGGATTGGGAAGACAATAGCAGGCATGCTGGGGATGGGGTGGGCTCTATGG

GS1196-016 expression cassette sequence SEQ ID NO: 15)

AGGCTCAGAGGCACACAGGAGTTTCTGGGCTCACCCTGCCCCCTTCCAACCCCTCAGTTCCCATCCTCCAGCAGCTGTTTGT
GTGCTGCCTCTGAAGTCCACACTGAACAAACTTCAGCCTACTCATGTCCCTAAAATGGGCAAACATTGCAAGCAGCAAAC
AGCAAACACACAGCCCTCCCTGCCTGCTGACCTTGGAGCTGGGGCAGAGGTCAGAGACCTCTCTGGGCCCATGCCACCTCC
AACATCCACTCGACCCCTTGGAATTTCGGTGGAGAGGAGCAGAGGTTGTCCTGGCGTGGTTTAGGTAGTGTGAGAGGGAG
GACGCCGCTGTTTACTGAGCTGGGCACAATGACCTTTGGCGAGCTGGACAGAGGGCCGGGCGCAGACGGGCAGGCGGGTG
GGCAGCTCGGCGCTGACCTTTGCCCTTAGTCCCTGTTTGCTCCTCCGATAACCGGGGTGACTTTGGTTAATCATTAACCAGC
AACCACCCCGTCGTCCCTGCGGGTCCACAGCTTAAATACGAACTCGAACGGGGCTCTGTCTCCTTAGCCAGGCACCACCA
CTGACCTGGGACAGTGAATCGTGAGTCTATGGGACCCTTGATGTTTTCTTTCCCCTTCTTTTCTATGGTTAAGTTCATGTCAT
AGGAAGGGGAGAAGTAACAGGGTACAGGGCTCGCGGTTGAGGACAAACTCTTCGCGGTCTTTCCAGTACTCTTGGATCGG
AAACCCGTCGGCCTCCGAACGGTACTCCGCCACCGAGGGACCTGAGCGAGTCCGCATCGACCGGATCGGAAAACCTCTCG
AGAAAGGCGTCTAACCAGTCACAGTCGCAAGGTAGGCTGAGCACCGTGGCGGGCGGCAGCGGGTGGCGGTCGGGGTTGTT
TCTGGCGGAGGTGCTGCTGATGATGTAATTAAAGTAGGCGGTCTTGAGACGGCGGATGGTCGAGGTGAGGTGTGGCAGGC
TTGAGATCCAGCTGTTGGGGTGAGTACTCCCTCTCAAAAGCGGGCATTACTTCTGCGCTAAGATTGTCAGTTCCAAAAAC
GAGGAGGATTTGATATTCACCTGGCCCGATCTGGCCATACACTTGAGTGACAATGACATCCACTTTGCCTTTCTCTCCACA
GGTGTCCACTCCCAGGTCCAAGTTTAAACTGTTCGGCTTCTGGCGTGTGACCGGCGGCTCTAGAGCCTCTGCTAACCATGTT
CATGCCTTCTTCTTTTTCCTACAGCTCCTGGGCAACGTGCTGGTTATTGTGCTGTCTCATCATTTTGGCAAGCCACCATGGCT
TCTAGGCTGACACTGCTGACACTGCTGCTGCTGCTGCTGGCTGGAGATAGAGCCTCCAGCAATCCCAATGCCACATCCAGC
TCCAGCCAAGACCCTGAGAGCCTCCAAGACAGAGGTGAGGGCAAAGTGGCCACAACTGTGATCTCCAAGATGCTCTTTGT
GGAGCCTATTCTGGAGGTGAGCTCTCTGCCCACCACCAATTCCACAACCAATTCTGCCACCAAGATCACTGCCAACACCAC
TGATGAGCCTACAACACAGCCTACAACAGAACCTACCACACAGCCCACCATTCAGCCCACCCAGCCCACCACACAGCTGC
CTACTGACAGCCCTACCCAGCCTACCACTGGCAGCTTCTGTCCTGGACCTGTGACACTGTGCTCTGATCTGGAGAGCCATA
GCACAGAGGCTGTGCTGGGTGATGCTCTGGTGGATTTCTCTCTGAAGCTGTATCATGCCTTCTCTGCCATGAAGAAGGTAG
AGACAAACATGGCCTTTAGCCCCTTCTCCATTGCTTCTCTGCTCACACAAGTGCTGCTGGGTGCTGGTGAGAATACCAAGA
CCAATCTGGAGTCCATCCTCTCCTACCCCAAGGACTTTACATGTGTGCACCAAGCCCTCAAGGGATTCACCACCAAGGGAG
TGACAAGTGTGAGCCAAATCTTCCACTCCCCTGACCTGGCCATCAGAGACACCTTTGTGAATGCCTCTAGGACACTGTACA
GCTCCTCCCCCAGAGTGCTGAGCAATAATAGTGATGCCAATCTGGAGCTCATCAATACATGGGTGGCTAAGAATACCAAC
AACAAGATCTCTAGGCTGCTGGATTCCCTCCCTTCTGACACAAGACTGGTGCTGCTGAATGCCATTTATCTGAGTGCCAAG
TGGAAGACCACATTTGACCCCAAGAAGACAAGAATGGAGCCCTTTCACTTCAAGAACTCTGTCATCAAGGTGCCCATGAT
GAACAGTAAGAAGTATCCTGTGGCCCACTTCATTGACCAGACCCTCAAGGCCAAAGTGGGACAGCTGCAGCTGAGCCACA
ATCTGTCTCTGGTGATTCTGGTGCCCCAGAATCTGAAACATAGACTGGAGGACATGGAACAAGCTCTGAGCCCCAGTGTCT
TTAAGGCCATCATGGAGAAGCTGGAGATGTCCAAGTTCCAGCCCACACTCCTCACACTGCCTAGAATCAAAGTCACCACA

AGCCAAGACATGCTGAGCATCATGGAAAAGCTGGAATTCTTTGACTTCAGCTATGATCTGAACCTCTGTGGACTCACAGAG
GATCCTGATCTGCAAGTGAGTGCCATGCAGCACCAGACTGTGCTGGAACTGACTGAAACTGGAGTGGAGGCTGCTGCTGC
CAGTGCTATCAGTGTGGCTAGAACACTGCTGGTGTTTGAGGTGCAGCAGCCCTTTCTGTTTGTCCTCTGGGATCAGCAGCA
TAAGTTCCCTGTGTTCATGGGAAGGGTGTATGATCCTAGAGCCTGACTGTGCCTTCTAGTTGCCAGCCATCTGTTGTTTGCC
CCTCCCCCGTGCCTTCCTTGACCCTGGAAGGTGCCACTCCCACTGTCCTTTCCTAATAAAATGAGGAAATTGCATCGCATTG
TCTGAGTAGGTGTCATTCTATTCTGGGGGGTGGGGTGGGGCAGGACAGCAAGGGGGAGGATTGGGAAGACAATAGCAGG
CATGCTGGGGATGGGGTGGGCTCTATGG

**Kozak sequence (SEQ ID NO: 16)**
GCCACCAUGG

**Materials, reagents, and consumables**

Mainly including HEK 293T cells; DMEM medium; C57-serpinG1$^{-/-}$ ; Human Serpin G1 ELISA Kit (Catalog No. EK1667, Wuhan Boster Biological Technology Co. Ltd), etc. Other materials, reagents, and consumables used, unless otherwise specified, are commercially available.

**Example 1. *In vitro* screening experiment for SerpinG1 codon optimization**

1. Vector construction: The inventors optimized the wild-type (WT) serpinG1 gene through codon optimization, generating three variants labeled as A, B, and C, respectively. Subsequently, the four genes, including the optimized A, B, C genes and the unoptimized wild-type serpinG1 gene (SEQ ID NO: 2), were individually cloned into the pcDNA3.1 backbone vector via seamless cloning, thereby yielding four expression vectors with FLAG tags: pcDNA3.1-serping1 optiA-3xFLAG-BGHpa, pcDNA3.1-serping1 optiB-3xFLAG-BGHpa, pcDNA3.1-serping1 optiC-3xFLAG-BGHpa, and pcDNA3.1-serping1 wt-3xFLAG-BGHpa.

2. Expression of serping1 and codon-optimized serping1 genes in cells: To test the expression levels of codon-optimized genes and the wild-type gene, HEK 293T cells were used for expression testing.

293T cells were inoculated in six-well cell culture plates. When the cell density reached 80% - 90%, the cells were transfected with the four expression plasmids. After 48 hours, cells were collected, and intracellular total protein was extracted. The BCA protein concentration assay kit (Catalog No. 20201ES76, Yeasen Biotechnology (Shanghai) Co., Ltd.) was used for quantification, followed by Western blot analysis with FLAG antibody (Monoclonal ANTI-FLAG® M2-Peroxidase (HRP) antibody, sigma) and SerpinG1 antibody (SERPING1 Antibody (OAAN00490), Aviva Systems Biology) to detect expression levels of each gene.

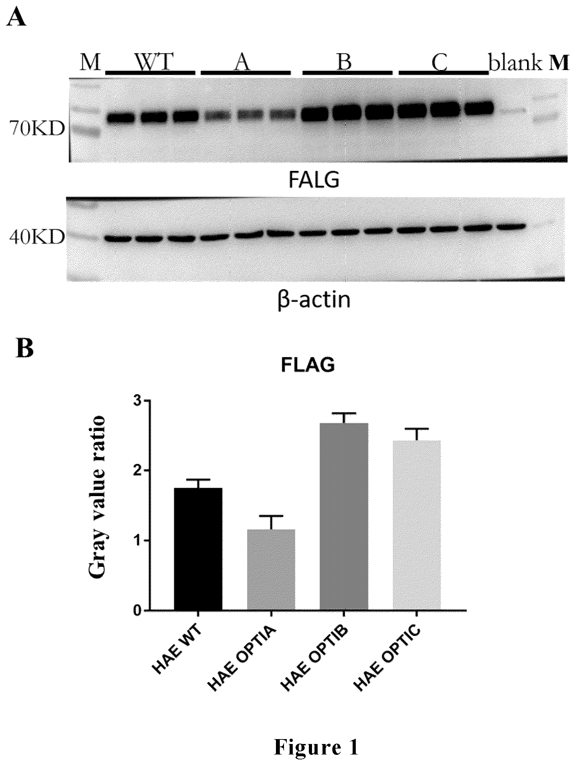

As shown in Figure 1 and Table 1, Western blot with FLAG antibody revealed the detected expression of codon-optimized A, B, C genes and the WT gene.

Table 1

|  | HAE WT | HAE optimized A | HAE optimized B | HAE optimized C |
|---|---|---|---|---|
|  | 68221 | 40341 | 93868 | 99986 |
| FALG gray value | 69584 | 41375 | 103357 | 101902 |
|  | 69682 | 48457 | 101962 | 88906 |
| FALG average gray value | 69162.33 | 43391 | 99729 | 96931 |
|  | 41877 | 38932 | 36060 | 40506 |
| Internal reference protein (β-actin) gray value | 39514 | 38908 | 36419 | 39580 |
|  | 37395 | 35141 | 39235 | 39486 |
| Internal reference protein average gray value | 39595.3 | 37660.33 | 37238 | 39857.33 |
|  | 1.62908 | 1.036191 | 2.603106 | 2.468424 |
| gray value ratio (FALG: β-actin) | 1.760996 | 1.063406 | 2.837997 | 2.574583 |
|  | 1.863404 | 1.378931 | 2.598751 | 2.251583 |
| average ratio | 1.75116 | 1.159509 | 2.679951 | 2.43153 |

According to the results of Figure 1A, Figure 1B, and Table 1, it is indicated that the expression levels of the codon-optimized B and C genes are superior to that of the wild-type serpinG1 gene. Among them, the expression level of the B gene is the best, reaching about 1.53 times that of the wild-type gene, and the expression level of the C gene is about 1.39 times that of the wild-type gene. However, the expression level of the codon-optimized A gene is lower than that of the wild-type serpinG1 gene.

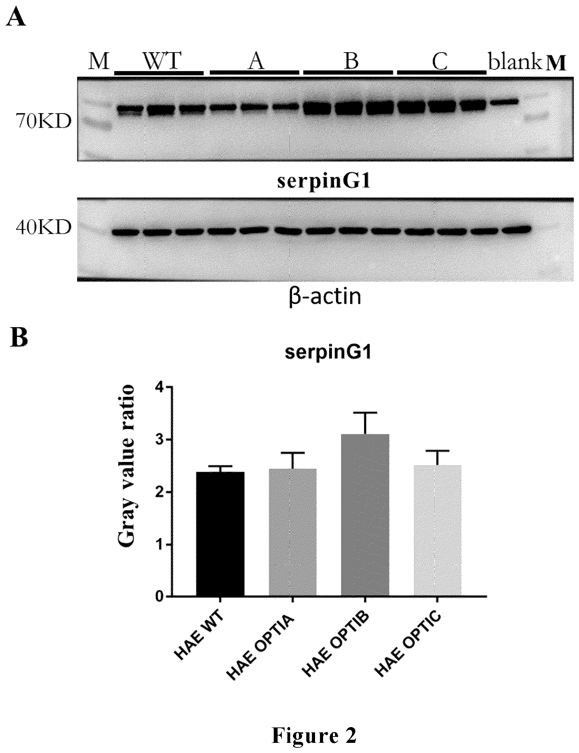

As shown in Figure 2 and Table 2, Western blot with SerpinG1 antibody revealed the detected expression of codon-optimized A, B, C genes and the WT gene.

Table 2

|  | HAE WT | HAE optimized A | HAE optimized B | HAE optimized C |
|---|---|---|---|---|
|  | 51650 | 50438 | 68337 | 69143 |
| serpinG1 gray value | 51772 | 49854 | 70540 | 68395 |
|  | 52113 | 48481 | 73454 | 58963 |
| FALG average gray value | 51845 | 49591 | 70777 | 65500 |
|  | 20713 | 21792 | 24924 | 24698 |
| Internal reference protein (β-actin) gray value | 21680 | 17856 | 23114 | 27389 |
|  | 22890 | 21614 | 20750 | 26121 |
| Internal reference protein average gray value | 21761 | 20420.67 | 22929.33 | 26069.33 |

(continued)

|  | HAE WT | HAE optimized A | HAE optimized B | HAE optimized C |
|---|---|---|---|---|
| gray value ratio (serpinG1: β-actin) | 2.493603 | 2.314519 | 2.741815 | 2.799538 |
|  | 2.388007 | 2.792003 | 3.05183 | 2.49717 |
|  | 2.276671 | 2.243037 | 3.539952 | 2.257303 |
| average ratio | 2.386094 | 2.449853 | 3.111199 | 2.518004 |

According to the results of Figure 2A, Figure 2B, and Table 2, it is indicated that all the expression levels of codon-optimized A gene, B gene, and C gene are superior to that of the wild-type serpinG1 gene. Among them, the expression level of the B gene is the best, reaching about 1.30 times that of the wild-type gene, while the expression levels of A gene and C gene are only slightly higher than that of the wild-type.

The above results all indicate that the expression level of the codon-optimized B gene (SEQ ID NO: 1) is the highest, reaching about 1.53 times that of the wild-type serpinG1 gene.

**Example 2. Detection of expression level of AAV expression vector comprising seping1 gene in model mice**

1. Construction of Y602 expression cassette:

The serpinG1 gene fragment (the codon-optimized B gene with the highest expression level was selected), poly (A) fragment (PA3), and various regulatory elements (including HCR, DSE, TPL, eMlp, sv40 intron, and optionally Kozak sequence) were seamlessly cloned together to obtain the expression cassette (5'-HCR-DSE-TPL-eM1p-sv40 intron-serping1-PA3-3'), that is, Y602 expression cassette.

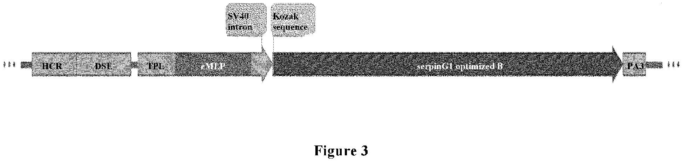

Figure 3 shows the structure of the Y602 expression cassette.

2. Packaging of the AAV vector: HEK293 cells were inoculated onto plates containing 10% FBS in DMEM medium at a concentration of $4 \times 10^6$/100 ml diameter, and cultured overnight in a humid environment containing 5% $CO_2$ at 37 °C. The next day, an AAV packaging plasmid containing the Y602 expression cassette and two inverted terminal repeat sequences (ITR) was constructed, and a PEI transfection mixture containing the nucleic acid molecule of the present application (SEQ ID NO: 1) or the nucleic acid molecule of the comparative example, AAV8 capsid protein, and auxiliary plasmid was prepared. Then, the transfection mixture was added to the cell culture medium and transfected for 6 hours. The medium was replaced with DMEM containing 10% FBS, and the cells were harvested after 72 hours of transfection to obtain a crude extract containing recombinant virus (recombinant AAV vector). Subsequently, the crude extract was resuspended in a buffer solution (pH=8) containing 100 mM sodium chloride, 2 mM magnesium chloride, and 10 mM Tris, and store at -80 °C.

3. Purification and quantitative analysis of the AAV vector: HEK-293 cells obtained in the previous step were subjected to three freeze-thaw cycles, treated with 50U/mL Benzonase at 37 °C for 30 minutes to remove unencapsulated DNA, centrifuged at 3,000g for 10 minutes to precipitate the cells, and the supernatant was transferred for ultracentrifugation.

An iodixanol centrifugation system was installed, four gradients of iodixanol solutions were sequentially added to the 33 ml Optiseal tube (Beckman) in the order of 17%, 25%, 40%, and 60% using a 10 ml syringe. Each solution was slowly added to the Optiseal tube from the bottom: 17% for 6 ml, 25% for 6 ml, 40% for 5 ml, and 60% for 4 ml. After addition, the sample name was labeled on the top of the Optiseal tube, and a line was marked at the boundary between 40% and 60%. Then, the supernatant was carefully added to the centrifuge tube, and centrifuged at 53,000g for 2 hours 40 minutes at 14 °C.

The needle of a 5-ml syringe was inserted along the previously marked horizontal line (the boundary between 40% and 60%) into the Optiseal tube, and 40% of the solution (approximately 2-3 ml) was aspirated into a new 15-ml tube. The virus solution was added to a pre-balanced 100K Centrifuge filter, topped up with $1 \times PBS$ ($10^{-4}$ F188) to near the 50-ml mark, and centrifuged at 3,500 rpm for 10 minutes. The waste liquid was discarded and the virus solution was refilled with $1 \times PBS$ ($10^{-4}$ F188) and centrifuged at 3,500 rpm for 10 minutes. This wash step was repeated for three times in total.

300-500 μl of $1 \times PBS$ ($10^{-4}$ F188) was added to collect the purified virus (i.e., recombinant AAV vector). The virus was transferred to a 1.5-ml EP tube. Subsequently, according to the kit instructions, qPCR was used to quantify the purified AAV vector genome and determine the titer of the original virus solution.

4. AAV vector expression and detection in mice: The obtained recombinant AAV vector was injected into the tail vein of serping1 model mice (including serpinG1-deficient mice serping1$^{-/-}$, and normal control (i.e., non-deficient) mice serping1$^{+/+}$) at a dose of 6E12 vg/kg. C1-INH levels in serum were measured at 1W and 2W post-injection using the Human Serpin G1 ELISA Kit (Catalog No. EK1667, Wuhan Boster).

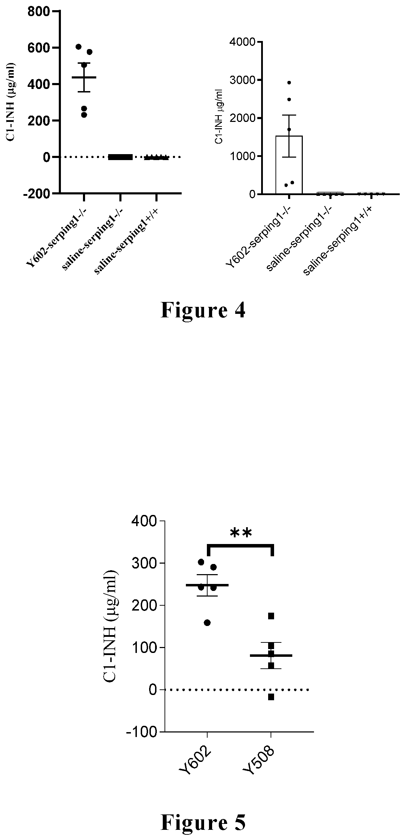

As shown in Figure 4, C1-INH levels in serping1 model mice at 1W and 2W post-injection of the AAV vector were revealed, with normal saline-injected mice as controls. Notably, after injection of the AAV vector, high levels of C1-INH were obviously detected in the plasma of mice, and the expression of C1-INH was sustained over time.

As shown in Figure 5 and Table 3, the *in vivo* expression effects of comparing the Y602 expression cassette with the Y508 expression cassette (LP1-SERPING1-PA3, another expression cassette constructed by the inventors in the same batch) were displayed (i.e., only the expression cassette was replaced, and all other experimental steps were the same). According to the difference in C1-INH content, it was found that there was a significant difference (p<0.01) in the corresponding C1-INH content between the Y602 expression cassette and the Y508 expression cassette. On average, the expression level of the Y602 expression cassette was about 3 times that of the Y508 expression cassette, with a maximum of approximately 4 times.

Table 3

| μg/ml | n1 | n2 | n3 | n4 | n5 | AVE |
|---|---|---|---|---|---|---|
| Y602 | 158.9 | 242.2 | 290.5 | 243.4 | 302.8 | 247.6 |
| Y508 | 174.7 | LOD | 104.5 | 57.1 | 85.3 | 81.0 |

The above results demonstrate that the Y602 expression cassette can be highly expressed and continuously expressed *in vivo,* and it has significantly better expression ability compared with other expression cassettes constructed in the same batch by the inventors (such as the Y508 expression cassette). After screening, the preferred expression cassette of the present invention is the Y602 expression cassette.

**Example 3. Expression detection of AAV expression vector comprising seping1 gene in cynomolgus monkeys**

1. Construction of GS1196-016 expression cassette:

In the present invention, Y602 was further optimized by combining the serpinG1 gene fragment (the codon-optimized B gene with the highest expression level was selected), poly (A) fragment (BGH), and various regulatory elements (including HCR2, DSE, TPL2, eMlp, HBB2 intron, and optionally Kozak sequence) through seamless cloning to obtain the expression cassette (5'-HCR2-DSE-TPL2-eM1p-HBB2 intron-serping1-BGH-3'), i.e. the GS1196-016 expression cassette.

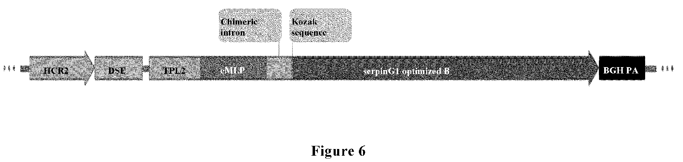

Figure 6 shows the structure of the GS 1196-016 expression cassette.

2. Packaging and quantification of the AAV vector. the virus packaging and quantification method in Example 2 was adopted in this example.

*3. In vivo* expression and detection of the AAV vector in cynomolgus monkeys (non-human primates, NHP model): Two male cynomolgus monkeys were selected as the experimental group, and GS1196-016 was administered intravenously at one dose of $1\times10^{13}$ vg/kg on Day 0. Plasma was collected on Day 7 to test the activity of C1-INH.

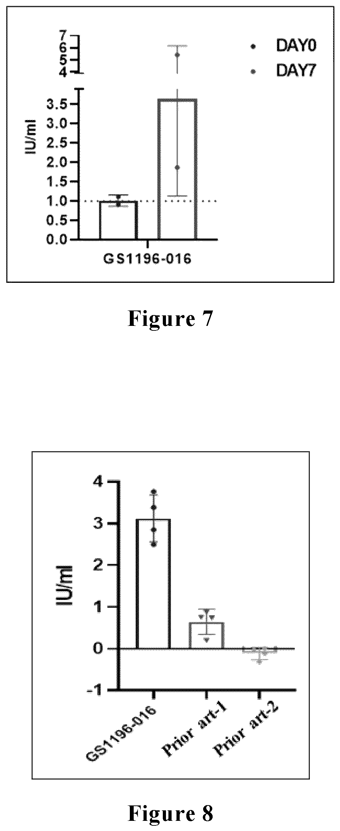

As shown in Figure 7, the C1-INH activity in cynomolgus monkeys at 1W post-injection of the AAV vector was displayed. Notably, after injection of the AAV vector, high levels of C1-INH activity were obviously detected in the plasma of cynomolgus monkeys, reaching and exceeding the clinically effective level. Therefore, the GS1196-016 expression cassette is also selected as the preferred expression cassette in the present application.

**Comparative experiment: Comparison of the expression of the expression cassette of the present invention with other expression cassettes mediating the same protein in mice**

In this example, the AAV vector construction method in Example 2, as well as the virus packaging and quantification method in Example 2 were adopted. The GS1196-016 expression cassette between the ITRs was replaced with other expression cassettes in the prior art, such as the preferred expression cassette in prior art-1 (selected from CN114829391A, with an expression cassette structure of 5'-ApoE/HCR enhancer-hAAT promoter-hhI, hAAT/hemoglobin intron-serping1-wt-hGH PA-3'), or prior art-2 (selected from US20230043051A1, with an expression cassette structure of 5'-3xCRM8-hTTR-MVM intron-serping1 HA06-wpre3-hBHG-3'), and subsequently packaged as AAV8 virus. Each AAV virus was administered via tail vein to 4 mice at a dose of $1\times10^{12}$ vg/kg on Day 0. Plasma samples were collected at Day 0 and Day 7 to test the activity of C1-INH.

As shown in Figure 8, the results indicate that using the expression cassette of the present invention to mediate the expression of C1-INH in mice is significantly more effective than other expression cassettes.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

**Claims**

1. An expression cassette having a structure shown in formula I from the 5' to 3' end:

$$Z1\text{-}Z2\text{-}Z3\text{-}Z4\text{-}Z5\text{-}Z6\text{-}Z7\text{-}Z8 \quad (I)$$

wherein each "-" is independently a bond or a nucleotide linker sequence;
Z1 is an HCR element;
Z2 is a DSE element;
Z3 is a TPL element;
Z4 is an eMlp element;
Z5 is an intron element;
Z6 is absent or is a Kozak sequence;
Z7 is a target gene; and
Z8 is a poly(A) element.

2. The expression cassette of claim 1, wherein the target gene is selected from the group consisting of: serpinG1 gene, FIX gene, PAH (phenylketonuria), GBA1 gene (Gaucher's disease), GLA gene (Fabry disease), IDS (mucopolysaccharidosis type II), G6P (favism), GAA (Pompe disease), luciferase gene, CFTR gene (cystic fibrosis), LDLR gene (familial hypercholesterolemia), $\alpha$-globin gene, $\beta$-globin gene (thalassemia), APC gene (familial adenomatous polyposis), SLC26A4 gene, GJB2 gene (congenital deafness), TYR gene, OCA2 gene, TYRP1 gene, SLC45A2 gene (albinism), and a combination thereof.

3. The expression cassette of claim 1, wherein the expression cassette has a nucleotide sequence as set forth in SEQ ID NO: 3 or SEQ ID NO: 15.

4. A nucleic acid molecule encoding the expression cassette of any one of claims 1-3.

5. An expression vector comprising the nucleic acid molecule of claim 4 or the expression cassette of any one of claims 1-3.

6. A host cell comprising the expression vector of claim 5 or having the nucleic acid molecule of claim 4 integrated into its genome.

7. A gene delivery system, comprising: the expression cassette of any one of claims 1-3 or the nucleic acid molecule of claim 4, and an AAV capsid protein.

8. Use of the expression cassette of any one of claims 1-3, the nucleic acid molecule of claim 4, the expression vector of claim 5, the host cell of claim 6, or the gene delivery system of claim 7, in the manufacture of a formulation or composition for use as a gene therapy medicament.

9. A pharmaceutical composition, comprising:

(i) the expression cassette of any one of claims 1-3, the nucleic acid molecule of claim 4, the expression vector of claim 5, the host cell of claim 6, or the gene delivery system of claim 7, as an active ingredient; and
(ii) a pharmaceutically acceptable carrier, diluent, or excipient.

10. A gene therapy method, comprising a step of: administering a therapeutically effective amount of the expression vector of claim 5, the gene delivery system of claim 7, or the pharmaceutical composition of claim 9, to a subject in need thereof.

11. A nucleic acid molecule encoding the serpinG1 gene, the nucleotide sequence of which has at least 87% identity, preferably at least 88%, 89%, 90%, 91%, 92%, 93%, 94%, 95%, 96%, 97%, 98%, 99%, or 100% identity to the sequence as set forth in SEQ **ID** NO: 1.

12. An expression vector comprising the nucleic acid molecule of claim 11.

13. A host cell comprising the expression vector of claim 12, or having the nucleic acid molecule of claim 11 integrated into its genome.

14. Use of the nucleic acid molecule of claim 11, the expression vector of claim 12, or the host cell of claim 13, in the manufacture of a formulation or composition for the treatment of hereditary angioedema.

15. A pharmaceutical composition, comprising:

(i) the nucleic acid molecule of claim 11, the expression vector of claim 12, or the host cell of claim 13, as an active ingredient; and
(ii) a pharmaceutically acceptable carrier, diluent, or excipient.

**A**

FALG

β-actin

**B**

FLAG

Figure 1

**A**

serpinG1

β-actin

**B**

serpinG1

**Figure 2**

| SV40 intron | Kozak sequence |

| HCR | DSE | TPL | eMLP | serpinG1 optimized B | PA3 |

**Figure 3**

**Figure 4**

**Figure 5**

Figure 6

**Figure 7**

**Figure 8**

## INTERNATIONAL SEARCH REPORT

| International application No. |
| --- |
| **PCT/CN2023/128560** |

| A. | CLASSIFICATION OF SUBJECT MATTER |
| --- | --- |

C12N 15/12(2006.01)i;  A61K 48/00(2006.01)i;  C12N 15/90(2006.01)i

According to International Patent Classification (IPC) or to both national classification and IPC

| B. | FIELDS SEARCHED |
| --- | --- |

Minimum documentation searched (classification system followed by classification symbols)

IPC: C12N, A61K

Documentation searched other than minimum documentation to the extent that such documents are included in the fields searched

Electronic data base consulted during the international search (name of data base and, where practicable, search terms used)

CNABS, DWPI, WPABSC, CNTXT, ENTXTC, ENTXT, OETXT, WPABS, VCN, VEN, CNKI, 百度学术搜索, Baidu Scholar Search, WEB OF SCIENCE, PubMed, GenBank, EMBL, 中国专利生物序列检索系统, China Patent Biological Sequence Search System: 表达盒, 核酸, 宿主细胞, 基因递送, HCR, DSE, TPL, eMlp, Kozak, SERPING1蛋白, serine protease inhibitor, clade G, member 1, C1-INH, Expression Cassettes, Nucleic Acids, Host Cells, Gene Delivery, 基于SEQ ID NO: 1-15的序列检索, search based on SEQ ID NOs: 1-15

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2022165027 A2 (SPARK THERAPEUTICS INC.) 04 August 2022 (2022-08-04) see abstract, and claims 1-61 | 1-15 |
| A | CN 113366106 A (CRISPR THERAPEUTICS AG et al.) 07 September 2021 (2021-09-07) see abstract, claims 1-114, and description, SEQ ID NO:106 | 1-15 |
| A | WO 2022226183 A2 (THE UNIVERSITY OF TEXAS SYSTEM) 27 October 2022 (2022-10-27) see abstract, and claims 1-74 | 1-10 |
| A | CN 110244048 A (EIGHTH MEDICAL CENTER OF PLA GENERAL HOSPITAL) 17 September 2019 (2019-09-17) see abstract, claims 1-10, and description, SEQ ID NO:6 | 11-15 |
| A | CA 3145112 A1 (REGENXBIO INC.) 04 February 2021 (2021-02-04) see abstract | 1-15 |

| ✓ Further documents are listed in the continuation of Box C. | ✓ See patent family annex. |
| --- | --- |

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **30 January 2024** | **06 February 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/128560** |

| C. | DOCUMENTS CONSIDERED TO BE RELEVANT | |
|---|---|---|
| Category* | Citation of document, with indication, where appropriate, of the relevant passages | Relevant to claim No. |
| A | WO 2022196245 A1 (CHROMOCENTER INC.) 22 September 2022 (2022-09-22)<br>see abstract | 1-15 |

Form PCT/ISA/210 (second sheet) (July 2022)

## INTERNATIONAL SEARCH REPORT

| International application No. |
|---|
| **PCT/CN2023/128560** |

---

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

---

Form PCT/ISA/210 (continuation of first sheet) (July 2022)

**INTERNATIONAL SEARCH REPORT**

International application No.

**PCT/CN2023/128560**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **10**
   because they relate to subject matter not required to be searched by this Authority, namely:

   The in vivo method involved in claim 10 falls within treatment methods implemented on the human/animal body (PCT Rule 39.1(iv)); however, the examiner still performs a search on the basis of "the use of a therapeutically effective amount of the expression vector of claim 5, the gene delivery system of claim 7, or the pharmaceutical composition of claim 9 in the preparation of a gene therapy drug".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/128560**

| Patent document cited in search report | | | Publication date (day/month/year) | Patent family member(s) | | | Publication date (day/month/year) |
|---|---|---|---|---|---|---|---|
| WO | 2022165027 | A2 | 04 August 2022 | AU | 2022214192 | A1 | 27 July 2023 |
| | | | | AU | 2022214192 | A9 | 24 August 2023 |
| | | | | IL | 304404 | A | 01 September 2023 |
| | | | | CA | 3207268 | A1 | 04 August 2022 |
| | | | | EP | 4284417 | A2 | 06 December 2023 |
| | | | | WO | 2022165027 | A3 | 09 September 2022 |
| | | | | KR | 20230136147 | A | 26 September 2023 |
| | | | | CO | 2023011154 | A2 | 08 September 2023 |
| CN | 113366106 | A | 07 September 2021 | EP | 3867377 | A1 | 25 August 2021 |
| | | | | JP | 2022505173 | A | 14 January 2022 |
| | | | | MX | 2021004455 | A | 11 August 2021 |
| | | | | AU | 2019362000 | A1 | 20 May 2021 |
| | | | | IL | 282369 | A | 30 June 2021 |
| | | | | CA | 3116885 | A1 | 23 April 2020 |
| | | | | US | 2021348159 | A1 | 11 November 2021 |
| | | | | WO | 2020081843 | A1 | 23 April 2020 |
| | | | | KR | 20210096088 | A | 04 August 2021 |
| WO | 2022226183 | A2 | 27 October 2022 | WO | 2022226183 | A3 | 01 December 2022 |
| CN | 110244048 | A | 17 September 2019 | | None | | |
| CA | 3145112 | A1 | 04 February 2021 | JP | 2022544004 | A | 17 October 2022 |
| | | | | IL | 289922 | A | 01 March 2022 |
| | | | | US | 2023042103 | A1 | 09 February 2023 |
| | | | | WO | 2021021661 | A1 | 04 February 2021 |
| | | | | EP | 4004214 | A1 | 01 June 2022 |
| WO | 2022196245 | A1 | 22 September 2022 | JPWO | 2022196245 | A1 | 22 September 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6566118 B
- US 6989264 B
- US 6995006 B
- US 2005027091 W
- WO 9118088 A
- WO 9309239 A
- US 4797368 A
- US 6596535 B
- US 5139941 A
- EP 0488528 A
- CN 114829391 A
- US 20230043051 A1

**Non-patent literature cited in the description**

- **LU et al.** *Molecular Therapy*, 2013, vol. 21 (5), 954-63
- **LU et al.** *Human Gene Therapy*, 2017, vol. 28 (1), 125-34
- **SAMBROOK et al.** Molecular Cloning: A Laboratory Manual. Cold Spring Harbor Laboratory Press, 1989